# EUROPÄISCHE PATENTSCHRIFT

(11) **EP 2 764 817 B1**
(45) Veröffentlichungstag und Bekanntmachung des Hinweises auf die Patenterteilung: **10.01.2018**
(21) Anmeldenummer: 14154065.8
(22) Anmeldetag: 06.02.2014
(51) Int. Cl.: A61B 1/00, A61B 1/005, A61M 25/01, A61B 17/00

(54) **Endoskopisches Instrument**
Endoscopic instrument
Instrument endoscopique

(30) Priorität: 07.02.2013 DE 102013101202
(43) Veröffentlichungstag der Anmeldung: 13.08.2014
(73) Patentinhaber: Karl Storz SE & Co. KG, 78532 Tuttlingen (DE)
(72) Erfinder: Schwarz, Peter, 78532 Tuttlingen-Nendingen (DE); Friedrich, Christina, 72184 Eutingen (DE)
(74) Vertreter: Witte, Weller & Partner Patentanwälte mbB

(56) Entgegenhaltungen:
- EP-A1- 2 039 284
- JP-A- 2005 028 018
- US-A- 4 499 895

## Beschreibung

Die vorliegende Erfindung betrifft ein endoskopisches Instrument mit einem Bedienstück mit einem Bedienelement, ferner mit einem Instrumentenschaft mit einem betätigbaren Abschnitt, wobei der Instrumentenschaft mit dem Bedienstück verbunden ist, einem Stellelement, einem Zugelement, das mit dem Stellelement und dem Abschnitt des Instrumentenschafts mechanisch gekoppelt ist, so dass eine Verlagerung des Stellelements eine Betätigung des Abschnitts mittels einer Kraftübertragung über das Zugelement bewirken kann, und außerdem mit einem Aktuator, der mit dem Stellelement gekoppelt ist, so dass eine Betätigung des Aktuators eine Verlagerung des Stellelements durch Kraftübertragung vom Aktuator auf das Stellelement bewirken kann.

Ein solches Endoskop ist aus US 4,499,895 bekannt.

Endoskopische Instrumente mit flexiblem oder starrem Instrumentenschaft werden sowohl in der Industrie als auch im medizinischen Bereich verwendet. Beispielsweise werden flexible Endoskope im veterinärmedizinischen Bereich bei der gastroendoskopischen Untersuchung von Großtieren eingesetzt. Diese Endoskope besitzen häufig an einem distalen Ende ihres Instrumentenschafts einen distalen Endabschnitt, der in einem Endstück endet. Das Endstück stellt den distalen Teil des Endoskops dar, der in den zu untersuchenden Körper eingeführt wird. Es weist üblicherweise das distale Ende einer Endoskopoptik sowie zum Teil auch Absaug-, Spül- und Werkzeugkanäle auf.

Um eine größtmögliche Flexibilität bezüglich einer räumlichen Ausrichtung des Endstücks bei der Untersuchung gewährleisten zu können, ist der Endabschnitt des Instrumentenschafts üblicherweise ablenkbar ausgebildet. Durch ein Ablenken, bzw. allgemein Betätigen, des Abschnitts zu dem restlichen Instrumentenschaft, insbesondere durch ein Abwinkeln oder Abkrümmen, kann ein Teil des Instrumentenschafts, insbesondere das Endstück, nach Wunsch ausgerichtet werden. Bei diesem Ablenken ist eine vorsichtige Vorgehensweise eines Anwenders notwendig, damit kein Gewebe verletzt wird, das sich um den Endabschnitt herum befindet. Es ist daher wichtig, dass das Betätigen des Abschnitts sehr präzise steuerbar ist.

Die Ablenkung des Abschnitts erfolgt bei flexiblen und starren Endoskopen gemäß dem Stand der Technik über ein Zugelement, insbesondere über Bowdenzüge. Das Zugelement ist mit einem Stellelement, insbesondere einem Lenkgetriebe, verbunden. Das Zugelement ist dabei häufig an einem Schnurrad befestigt. Durch eine Betätigung des Bedienelements am Bedienstück des Endoskops wird das Stellelement bewegt. Durch eine rotatorische Bewegung des Stellelements ergibt sich eine translatorische Bewegung des Zugelements. Die Bewegung des Zugelements hat wiederum eine Ablenkung des Abschnitts zur Folge.

Die eingangs genannte US 4,499,895 zeigt ein elektrisches Endoskop, dessen Bedienung über einen Hebel erfolgt, der mit dem Stellelement gekoppelt ist. Wenn der Hebel gegenüber dem Stellelement verlagert wird, wird eine Biegung des Hebels oder eine Widerstandsänderung eines Potentiometers erfasst und dadurch eine Unterstützung des Stellelements durch einen Aktuator eingestellt. Der gezeigte Aufbau ist allerdings sehr unhandlich und erfordert im Fehlerfall einen speziellen Lösemechanismus, um das Endoskop noch bedienen zu können.

US 7,331,924 beschreibt ein elektrisches Endoskop mit einem ablenkbaren distalen Abschnitt. Die Bedienung des Endoskops erfolgt hier über einen Trackball, dessen Verlagerung durch den Daumen oder einen anderen Finger eines Benutzers von einer elektrischen Schaltung erfasst wird. In Abhängigkeit von der rotatorischen Verlagerung des Trackballs, wie sie vom Benutzer gewählt wird, wird in einer Ablenkungssteuerung die Ansteuerung eines Motors bestimmt, der eine Verlagerung des Zugelements und damit ein Ablenken des distalen Abschnitts bewirkt.

Der Nachteil eines solchen elektrischen Endoskops ist jedoch, dass die Bedienung als weniger intuitiv empfunden wird, da die Bedienung eines elektrischen Endoskops, z.B. beim Anstoß an Gewebe, nicht die Rückmeldung in Form einer Gegenkraft am Bedienelement liefert, die der Benutzer von mechanischen Endoskopen kennt. Dieselbe Problematik ergibt sich auch bei elektrischen Endoskopen, die über einen Joystick bedient werden. Dabei sei lediglich beispielsweise auf das Dokument US 6,932,761 hingewiesen. Elektrische Endoskope haben außerdem den Nachteil, dass sie sich bei einem Defekt möglicherweise nur schwer aus dem Hohlraum herausziehen lassen, wenn der distale Abschnitt abgelenkt ist.

Bei mechanischen Endoskopen erfolgt die Ablenkung des Abschnitts ausschließlich über eine mechanische Krafteinwirkung des Benutzers auf eine außen liegende Handhabe am Bedienstück des Endoskops. Dafür ist das Stellelement üblicherweise fest auf einer Welle der Handhabe angeordnet. Betätigt der Benutzer die Handhabe, so entsteht eine rotatorische Verlagerung des Stellelements und dadurch wiederum die translatorische Bewegung des Zugelements.

Rein mechanische Endoskope können aber je nach Länge des Endoskops und der Lage des Instrumentenschafts erhebliche Kräfte für eine Betätigung erfordern. Zudem erzeugt die mechanische Betätigung des Abschnitts automatisch eine gewisse Rückstellkraft in Richtung der nichtabgelenkten Position (Nullposition) des Endoskops. Ferner bildet der Abschnitt bei der Ablenkung mit dem Zugelement ein FederDämpfer-System, das Energie beim Spannen speichert und beim Entspannen freigibt. Dadurch können eine Anlauftotzeit oder ein Nachlaufweg beim Ablenken des Endabschnitts entstehen. Dies führt dazu, dass der Benutzer die Ablenkung des Abschnitts, die er eigentlich einstellen möchte, um eine bestimmte Stelle betrachten zu können, nur näherungsweise oder iterativ einstellen kann.

Es ist daher eine Aufgabe der vorliegenden Erfindung, ein endoskopisches Instrument der eingangs genannten Art dahingehend weiterzubilden, dass der Benutzer zwar eine Unterstützung, wie bei einer elektrischen Betätigung, bei der Bedienung erhält, dabei aber nicht auf eine mechanische Rückmeldung durch das Endoskop verzichten muss. Außerdem ist es eine Aufgabe, dass das Endoskop selbst bei einem Ausfall der elektrischen Steuerung weiterhin bedienbar bleibt.

Die Aufgabe wird gelöst durch ein eingangs genanntes endoskopisches Instrument mit einem Zwischenelement, das mit dem Bedienelement und dem Stellelement wirkverbunden ist, so dass eine vom Benutzer auf das Bedienelement ausgeübte Kraft über das Zwischenelement auf das Stellelement übertragen werden kann, um das Stellelement zu verlagern, einer Erfassungseinrichtung zum Erfassen einer Torsion des Zwischenelements, und einer Anschlagseinrichtung, die einen freien rotatorischen Bewegungsbereich des Bedienelements relativ zum Stellelement definiert.

Die Erfindung bietet mehrere Besonderheiten, die insbesondere in der Ausgestaltung des Zwischenelements und der Anschlagseinrichtung und im Zusammenspiel von Bedienelement, Zwischenelement und Stellelement gesehen werden. Um dies zu verdeutlichen, werden nachfolgend die verschiedenen Betätigungsmöglichkeiten des endoskopischen Instruments erläutert.

Als erstes soll die Möglichkeit eines rein motorischen Betriebs betrachtet werden. Dabei betätigt ein Benutzer das Bedienelement derart, dass sich das Bedienelement geringfügig gegenüber dem Stellelement verlagert, insbesondere rotatorisch verlagert. Die Kraft des Benutzers wird vom Bedienelement auf das Zwischenelement übertragen. Da das Zwischenelement mit dem Stellelement wirkverbunden ist, insbesondere direkt verbunden ist, und sich das Stellelement zunächst nicht bewegt, resultiert eine Torsion bzw. Verdrehung des Zwischenelements, zumindest eines Abschnitts des Zwischenelements.

Es sei an dieser Stelle darauf hingewiesen, dass der Begriff "Kraft" auch ein Drehmoment umfassen soll, da sich dieses insbesondere aus der Länge eines Hebelarms multipliziert mit einer Kraft ergibt.

Die Torsion des Zwischenelements kann von der Erfassungseinrichtung erfasst werden. Dabei ist es vorteilhaft, wenn nicht nur alleine das Vorhandensein einer Torsion ermittelt wird, sondern auch die Richtung der Torsion, also ob es sich um eine Torsion im Uhrzeigersinn oder gegen den Uhrzeigersinn handelt. Besonders bevorzugt ist es, wenn auch die Stärke der Torsion ermittelt wird, da dies einen Rückschluss auf die vom Benutzer aufgebrachte Kraft ermöglicht.

Aus der Kenntnis der Torsion kann auf die vom Benutzer durchgeführte Betätigung geschlossen werden. Dabei kann insbesondere die vom Benutzer gewählte Betätigungsrichtung und/oder die vom Benutzer aufgebrachte Kraft erkannt werden. Da somit die vom Benutzer gewünschte Betätigung des endoskopischen Instruments erkannt wird, wird dann der Aktuator derart angesteuert, dass er die vom Benutzer gewünschte Verlagerung des Stellelements vornimmt. Der Aktuator steht dafür in Wirkverbindung, bevorzugt in direkter Verbindung und besonders bevorzugt in direkter mechanischer Verbindung zum Stellelement. Der Benutzer bedient das endoskopische Instrument dann rein motorisch.

Bei einer weiteren Betriebsmöglichkeit wird das Stellelement durch eine Kombination von motorischer Betätigung und durch die Kraft des Benutzers bewirkt. Dabei gilt zunächst wieder, dass eine Torsion des Zwischenelements, zumindest eines Abschnitts des Zwischenelements, zu einer motorischen Unterstützung führt. Nun kann es allerdings sein, dass der Benutzer eine so große Kraft aufbringt, dass der freie rotatorische Bewegungsbereich des Bedienelements relativ zum Stellelement ausgeschöpft wird.

Dies bedeutet, dass das Bedienelement die Anschlagseinrichtung mechanisch kontaktiert. Dann findet eine direkte Übertragung eines Teils der Kraft des Benutzers über die Anschlagseinrichtung auf das Stellelement statt. Damit erfolgt die Verlagerung des Stellelements sowohl durch den Aktuator als auch durch zumindest einen Teil der vom Benutzer aufgebrachten Kraft. Außerdem kann so verhindert werden, dass die Kraft des Benutzers zu einer plastischen Verformung des Zwischenelements oder eines Messelements der Erfassungseinrichtung führt, was eine Beschädigung zur Folge hätte. Die Anschlagseinrichtung stellt also auch einen Überlastschutz dar.

Schließlich soll der Fall einer Betätigung betrachtet werden, die ausschließlich durch die Kraft des Benutzers erfolgt, also rein manuell. Dabei wird angenommen, dass die motorische Unterstützung durch den Aktuator entfällt. Der Grund hierfür kann ein Ausfall des Aktuators oder seiner Ansteuerung sein oder, wie es bei einer bevorzugten Ausführungsform möglich ist, weil der Benutzer die motorische Unterstützung abgeschaltet hat. Bei dieser Betriebsmöglichkeit findet weiterhin eine Torsion des Zwischenelements statt, jedoch keine Verlagerung des Stellelements durch den Aktuator. Daher ist es erforderlich, dass die Anschlagseinrichtung eine Kraftübertragung vom Bedienelement auf das Stellelement bereitstellt, um die Kraft des Benutzers zumindest zum größten Teil auf das Stellelement zu übertragen. Dies erfolgt dadurch, dass der freie rotatorische Bewegungsbereich des Bedienelements relativ zum Stellelement ausgeschöpft wird. Dann findet eine direkte Übertragung zumindest des größten Teils der Kraft des Benutzers über die Anschlagseinrichtung auf das Stellelement statt

Wie anhand der Ausführungsbeispiele noch verdeutlicht wird, wird es als vorteilhaft angesehen, wenn die Anschlagseinrichtung durch zwei Vorsprünge auf dem Stellelement realisiert ist und sich ein Abschnitt des Bedienelements zwischen diesen zwei Vorsprüngen bewegt oder an diesen anliegt. Bei einer anderen vorteilhaften Ausgestaltung ist die Anschlagseinrichtung als Ausnehmung oder Nut in dem Stellelement ausgebildet und der Abschnitt greift mit einem Vorsprung in diese Ausnehmung bzw. diese Nut ein. Bei einer weiteren vorteilhaften Ausgestaltung ist die Anschlagseinrichtung als ein Vorsprung auf dem Stellelement angeordnet und ist das Ende des Abschnitts in der Art einer Gabel mit zwei Zinken ausgebildet, wobei der Vorsprung zwischen den Zinken angeordnet ist. Schließlich ist bei einer weiteren vorteilhaften Ausgestaltung die Anschlagseinrichtung als ein Vorsprung auf dem Stellelement angeordnet, der in eine Ausnehmung oder Nut des Abschnitts eingreift. Es ist vorteilhaft, wenn der Abschnitt direkt mit dem Bedienelement verbunden ist, insbesondere einstückig ausgeführt ist.

Es sei darauf hingewiesen, dass das Stellelement mehrere Bestandteile aufweisen kann, insbesondere Verbindungselemente und Kraftübertragungselemente wie zum Beispiel Achsen, Scheiben oder Zahnräder. Bei einer bevorzugten Ausführungsform weist das Stellelement ein Schnurrad auf. Bei einer weiteren bevorzugten Ausführungsform weist das Stellelement zudem ein Getriebe auf, das eine vom Zwischenelement auf das Stelleelement übertragene Kraft auf das Schnurrad überträgt

Bei dem Zwischenelement kann es sich um ein von Bedienelement und Stellelement separates Element handeln. Bei bestimmen Ausführungsformen kann das Zwischenelement auch einstückig mit dem Bedienelement oder mit dem Stellelement ausgebildet sein. Grundsätzlich ist es zudem möglich, Bedienelement, Stellelement und Zwischenelement einstückig auszubilden. Bei einer bevorzugten Ausführungsform fällt eine Längserstreckung des Zwischenelements mit einer Rotationsachse des Bedienelements und/oder einer Rotationsachse des Stellelements zusammen.

Damit ist die Aufgabe vollständig gelöst.

Bei einer bevorzugten Ausgestaltung der Erfindung ist ein Messelement der Erfassungseinrichtung auf dem Zwischenelement angeordnet.

Bei dieser Ausgestaltung lässt sich die Erfassungseinrichtung günstig realisieren. Beispielsweise kann das Messelement auf das Zwischenelement geklebt werden. Bevorzugt werden leitende Kunststoffe verwendet, die bei Bedarf in einen flexiblen, isolierenden Werkstoff eingebettet sind, die bei einer Dehnung eine Änderung des elektrischen Widerstands zeigen. Es ist besonders bevorzugt, das Messelement als spritzgegossenen Schaltungsträger (MID, molded interconnect devices) auf das Zwischenelement aufzubringen. Es ist auch möglich, das Messelement, gegebenenfalls einschließlich der Erfassungseinrichtung, in dem Zwischenelement anzuordnen, wo es gut geschützt ist.

Bei einer weiteren vorteilhaften Ausgestaltung der Erfindung weist die Erfassungseinrichtung ein Messelement auf, das derart ausgebildet ist, dass sich bei Änderung einer Form des Messelements der elektrische Widerstand des Messelements ändert.

Diese Ausgestaltung ermöglicht eine einfache messtechnische Erfassung der Torsion. Außerdem kann die Erfassungseinrichtung besonders kompakt ausgestaltet werden. Als Messelement wird bevorzugt ein Leiter oder eine Leiterbahn eingesetzt, der/die so angeordnet ist, dass sich seine/ihre Form bei einer Torsion des Zwischenelements ändert, insbesondere durch eine Biegung, Stauchung oder Streckung.

Bei einer weiteren vorteilhaften Ausgestaltung der Erfindung weist die Erfassungseinrichtung ein Messelement auf, das als Dehnungsmesstreifen ausgebildet ist.

Diese Ausgestaltung ermöglicht eine kostengünstige Realisierung der Erfassungseinrichtung, da hier bereits umfangreiche theoretische Erkenntnisse, praktische Erfahrungen und ein großes Produktangebot verfügbar sind. Lediglich beispielhaft sei auf die Veröffentlichung "Messtechnische Übungen II, Messen mit DMS-Aufnehmern", Dipl.-Ing. Johannes Thaten, Technische Universität Berlin, 2006, und die Veröffentlichung "Drehmomentwellenberechnung", Telemetrie-Messtechnik Schnorrenberg, 2009 hingewiesen.

Bei einer weiteren vorteilhaften Ausgestaltung der Erfindung weist die Erfassungseinrichtung mindestens zwei Messelemente auf, die derart angeordnet sind, dass in der Erfassungseinrichtung eine Kompensation von mindestens einem Störeinfluss ausgewählt aus der Gruppe bestehend aus Biegung, Längsstreckung und Temperatur ermöglicht ist.

Diese Ausgestaltung ermöglicht, dass der Betrieb des endoskopischen Instruments unabhängig oder zumindest weitestgehend unabhängig von Umgebungseinflüssen möglich ist. Bezüglich einer Temperaturkompensation sei lediglich beispielhaft auf die Veröffentlichung "Torsions-Aufnehmer mit DMS-1/2-Brückenanschluss für Temperaturkompensation und R-Abgleich", VISHAY Measurements Group Messtechnik GmbH, 1994 hingewiesen.

Bei einer weiteren vorteilhaften Ausgestaltung der Erfindung weist das Zwischenelement zumindest einen ersten Bereich und einen zweiten Bereich auf, wobei der erste Bereich aus einem anderen Material besteht als der zweite Bereich.

Diese Ausgestaltung ermöglicht es, den zweiten Bereich im Hinblick auf die gewünschte Torsionsmessung zu optimieren. Dabei werden für den zweiten Bereich bevorzugt solche Materialien verwendet, die mit geringer Kraft elastisch verformt werden können und gleichzeitig über einen weiten Bereich elastisch bleiben. Als besonders vorteilhaft werden hier Stoffe aus der Gruppe der Polyaryletherketone, insbesondere Polyetheretherketon (PEEK) angesehen. Alternativ oder zusätzlich kann auch der Querschnitt des zweiten Bereichs gegenüber dem ersten Bereich variiert werden, um eine gute Abstimmung zwischen Betätigungskraft, Hebelarm, Verformung und genügend genauem Messsignal im zur Verfügung stehenden Bauraum zu finden. Bevorzugt sind erster und zweiter Bereich entlang einer Längserstreckung des Zwischenelements hintereinander angeordnet.

Bei einer weiteren vorteilhaften Ausgestaltung der Erfindung besteht das Zwischenelement aus einem anderen Material als das Bedienelement und/oder das Stellelement.

Diese Ausgestaltung ermöglicht es, das Zwischenelement im Hinblick auf die gewünschte Torsionsmessung zu optimieren. Unabhängig von dieser Materialwahl können die Materialien für das Bedienelement und das Stellelement frei gewählt werden, insbesondere im Hinblick auf die Anforderungen, die an das Bedienelement und das Stellelement gestellt werden.

Bei einer weiteren vorteilhaften Ausgestaltung der Erfindung ist die Anschlagseinrichtung einstellbar, um den Bewegungsbereich zu variieren.

Auf diese Weise kann individuell eingestellt werden, wie weit eine freie rotatorische Verlagerung des Bedienelements gegenüber dem Stellelement möglich sein soll, bevor eine zumindest teilweise Kraftübertragung über die Anschlagseinrichtung auf das Stellelement erfolgt. Bei einigen Ausgestaltungen ist es vorteilhaft, den freien Bewegungsbereich relativ groß zu wählen, da dies eine rein motorische Steuerung in einem breiten Bereich ermöglicht. Bei anderen Ausgestaltungen ist es vorteilhaft, den freien Bewegungsbereich klein zu halten, so dass eine Kombination von motorischer Betätigung und Betätigung durch die Kraft des Benutzers frühzeitig erfolgt. Die Anschlagseinrichtung wird bevorzugt eingestellt, indem Anschlagselemente verwendet werden, bei denen die Position einer Anschlagsfläche veränderbar ist, insbesondere durch Drehen, Schrauben oder Schieben. Die Aussagen zu der Wahl der Größe des freien Bewegungsbereichs gelten genauso für den Fall, wenn die Anschlagseinrichtung nicht einstellbar ist.

Bei einer weiteren vorteilhaften Ausgestaltung der Erfindung weist das Stellelement ein Getriebe auf, insbesondere ein Getriebe mit einem positiven Übersetzungsverhältnis.

Diese Ausgestaltung ermöglicht eine besonders gute Anpassung, wie die vom Benutzer aufgebrachte Kraft auf das Zugelement übertragen wird. Dabei ist es besonders vorteilhaft, dass das Getriebe als Kombination von Außenzahnrad und Innenzahnrad realisiert ist. Dabei sei an dieser Stelle darauf hingewiesen, dass bei einer anderen bevorzugten Ausführungsform der Aktuator ein Getriebe aufweist.

Bei einer weiteren vorteilhaften Ausgestaltung der Erfindung fallen die erste Rotationsachse des Stellelements, eine zweite Rotationsachse des Bedienelements und eine Längssachse des Zwischenelements zusammen.

Diese Ausgestaltung ermöglicht einen kostengünstigen Aufbau und eine einfache Kraftübertragung vom Bedienelement auf das Zwischenelement, vom Zwischenelement auf das Stellelement und, wenn der Betrieb nicht rein motorisch ist, vom Bedienelement auf das Stellelement.

Bei einer weiteren vorteilhaften Ausgestaltung der Erfindung fallen eine erste Rotationsachse des Stelleelements und eine dritte Rotationsachse des Aktuators zusammen.

Diese Ausgestaltung ermöglicht einen kostengünstigen Aufbau und eine einfache Kraftübertragung des Aktuators auf das Stellelement. Insbesondere fallen auch eine Rotationsachse eines Schnurrads und die dritte Rotationsachse des Aktuators zusammen.

Bei einer weiteren vorteilhaften Ausgestaltung der Erfindung weist das Stellelement ein zumindest in etwa kreisförmiges Trägerelement auf, auf dem die Anschlagseinrichtung zumindest in etwa in Umfangsrichtung des Trägerelements angeordnet ist.

Diese Ausgestaltung ermöglicht eine kostengünstige Fertigung des endoskopischen Instruments und einen zuverlässigen Betrieb. Dabei ist es bei einigen Ausführungsformen bevorzugt, wenn das Trägerelement in direktem mechanischen Kontakt mit dem Zugelement steht und insbesondere das Trägerelement als Schnurrad ausgeführt ist. Bei anderen Ausgestaltungen ist es vorteilhaft, insbesondere bei Verwendung eines Getriebes, dass das Trägerelement nicht in direktem mechanischen Kontakt mit dem Zugelement steht, insbesondere das Trägerelement ein Bauteil separat vom Schnurrad ist.

Bei der Erfindung weist das endoskopische Instrument ferner eine Steuereinrichtung auf, die dafür ausgebildet ist, einen Messwert der Torsion aufzunehmen und den Aktuator unter Berücksichtigung des Messwerts anzusteuern.

Diese Ausgestaltung ermöglicht eine vorteilhafte Realisierung des rein motorischen bzw. des kombinierten Betriebs. Die Steuereinrichtung wertet im Zusammenspiel mit dem Erfassungselement einen Messwert oder ein Messsignal aus, das eine Torsion des Zwischenelements anzeigt. Dabei wird zumindest erkannt, dass der Benutzer das endoskopische Instrument betätigen möchte. Es ist bevorzugt, dabei ebenfalls die Richtung der gewünschten Betätigung zu ermitteln und insbesondere auch eine Information über die vom Benutzer aufgebrachte Kraft zu ermitteln. Die Steuereinrichtung erhält demnach eine Information darüber, dass der Benutzer das endoskopische Instrument betätigt und bevorzugt wie er es betätigt. Die Steuereinrichtung ist dafür ausgebildet, den Aktuator so anzusteuern, dass die vom Benutzer gewünschte Betätigung des endoskopischen Instruments motorisch vorgenommen oder zumindest unterstützt wird.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung näher dargestellt und werden in der nachfolgenden Beschreibung näher erläutert. Es zeigen:
- Fig. 1: ein Ausführungsbeispiel eines flexiblen Endoskops mit einem ablenkbaren distalen Abschnitt,
- Fig. 2: das Endoskop gemäß Fig. 1 in vereinfachter schematischer teilweiser Schnittdarstellung,
- Fig. 3: Bedienelement und Stellelement einer ersten Ausführungsform,
- Fig. 4: das Zusammenspiel von Bedienelement, Stellelement und Anschlagseinrichtung bei einer zweiten Ausführungsform im freien rotatorischen Bewegungsbereich des Bedienelements relativ zum Stellelement,
- Fig. 5: das Zusammenspiel von Bedienelement, Stellelement und Anschlagseinrichtung bei der zweiten Ausführungsform wenn der freie rotatorische Bewegungsbereich ausgeschöpft ist,
- Fig. 6: eine Anordnung von Bedienelement, Stellelement und Zwischenelement einer dritten Ausführungsform,
- Fig. 7: eine Anordnung von Bedienelement, Stellelement, Zwischenelement und Aktuator einer vierten Ausführungsform,
- Fig. 8: eine vereinfachte Darstellung des Getriebes gemäß der dritten Ausführungsform, und
- Fig. 9: eine Anordnung von Bedienelement, Stellelement, Zwischenelement und Aktuator einer fünften Ausführungsform.

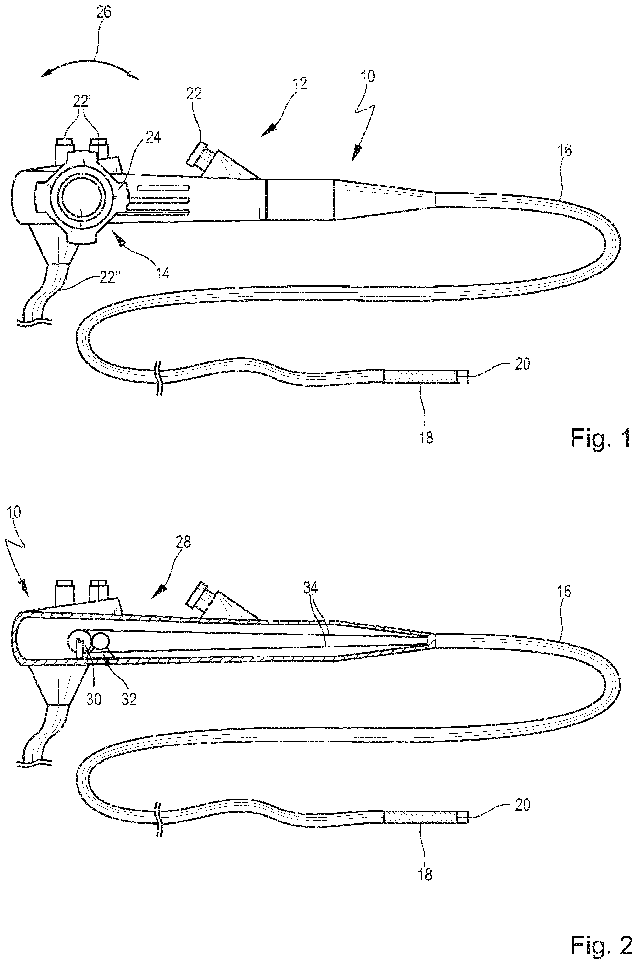

Fig. 1 zeigt ein endoskopisches Instrument 10 mit einem Bedienstück 12, mit einem Bedienelement 14 und einem flexiblen Instrumentenschaft 16. Es sei darauf hingewiesen, dass die Erläuterungen im Rahmen der Erfindung in gleicher Weise für einen starren Instrumentenschaft (nicht gezeigt) zutreffen.

Das endoskopische Instrument 10 wird zu Untersuchungs- und/oder Operationszwecken in medizinischen Verfahren verwendet. In dem Instrumentenschaft 16 verlaufen eine nicht dargestellte Endoskopoptik in Form von Lichtleitfasern, Bildleitern, verschiedene Kanäle, wie ein Saug- und ein Spülkanal, und ein Instrumentenkanal. Der Instrumentenschaft 16 ist proximal mit dem Bedienstück 12 verbunden und erstreckt sich distal bis in einen ablenkbaren Abschnitt 18, bei dem es sich hier insbesondere um einen Endabschnitt handelt.

Dieser weist ein Endstück 20 in Form einer Abschlussbuchse auf. Das Endstück 20 ist der Bereich des Instrumentenschafts 16, in dem die Lichtleitfasern, die Bildleiter und Kanäle enden. Der Instrumentenschaft 16 ist in den Figuren nur teilweise dargestellt. Das Bedienstück 12 weist einen Anschluss 22, Tasten 22' und ein Versorgungskabel 22" auf. Der Anschluss 22 führt zu einem Instrumentenkanal. Durch ihn können Instrumente in den Instrumentenschaft 16 und bis durch das Endstück 20 hindurch geführt werden. Dadurch können im Bereich vor dem Endstück 20 beispielsweise Operationen durchgeführt werden.

Das Versorgungskabel 22" beinhaltet verschiedene Arten von Versorgungen, wie beispielsweise eine elektrische Versorgung, Lichtleiter, Saug- und Spülschläuche und/oder Datenleitungen. Die Bildgebung des endoskopischen Instruments 10 erfolgt über einen nicht dargestellten Bildsensor im Inneren des Bedienstücks 12 oder im Endstück 20, wobei die Bilddaten über das Versorgungskabel 22" nach außen geleitet werden, insbesondere zu einer nicht dargestellten Kamerasteuerungseinheit (camera control unit, CCU).

Das Ablenken des Abschnitts 18 wird mittels des Bedienelements 14 gesteuert, wobei hier eine Ausführungsform mit einer Handhabe 24 zu sehen ist, die in die Richtungen des Doppelpfeils 26 gedreht werden kann. Durch Drehen des Bedienelements 14 wird der ablenkbare Abschnitt 18 auf/ab oder links/rechts abgelenkt. Die jeweilige Ablenkung korrespondiert hier mit der Drehrichtung des Bedienelements 14, entweder gegen oder im Uhrzeigersinn. Bei einer bevorzugten Ausführungsform wird zusätzlich zum gezeigten Bedienelement 14 ein nicht gezeigtes, weiteres Bedienelement verwendet, so dass über ein weiteres Stellelement und ein weiteres Zugelement Bewegungen auf/ab und links/rechts kombiniert werden können.

Wie in Fig. 2 stark vereinfacht dargestellt ist, sind in einem proximalen Endbereich 28 des Bedienstücks 12 ein Stellelement 30 und ein Aktuator 32 angeordnet. Das Stellelement 30 ist mit einem Zugelement 34 verbunden, das durch den Instrumentenschaft 16 hindurchgeführt ist und sich bis in den Endabschnitt 18 hinein erstreckt. Das Zugelement 34 ist um einen Teil des Stellelements 30 herum geführt und hier einstückig ausgebildet.

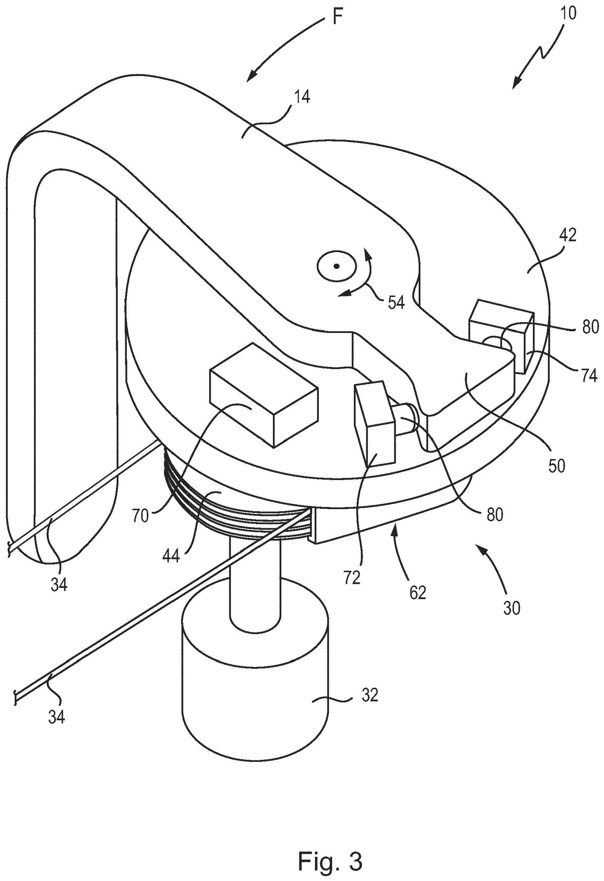

Fig. 3 zeigt ein erstes Ausführungsbeispiel eines Bedienelements 14 und eines Stellelements 30. Von dem Stellelement 30 ist hier ein zumindest in etwa kreisförmiges Trägerelement 42 und ein Schnurrad 44 zu erkennen. Das Bedienelement 14 ist dafür ausgebildet, dass es in eine Bewegungsrichtung 54 verlagert werden kann.

Das Bedienelement 14 ist mit dem Stellelement 30 wirkverbunden, so dass eine vom Benutzer auf das Bedienelement 14 ausgeübte Kraft F über eine Anschlagseinrichtung 62 auf das Stellelement 30 übertragen werden kann, um das Stellelement 30 zu verlagern, hier insbesondere das Schnurrad 44 zu verlagern.

Das Bedienelement 14 weist einen Abschnitt 50 auf. Der Abschnitt 50 ist hier einstückig mit dem Bedienelement 14 ausgebildet. Ein Zwischenelement 40 (hier nicht gezeigt) ist in dieser Ansicht vom Bedienelement 14 verdeckt.

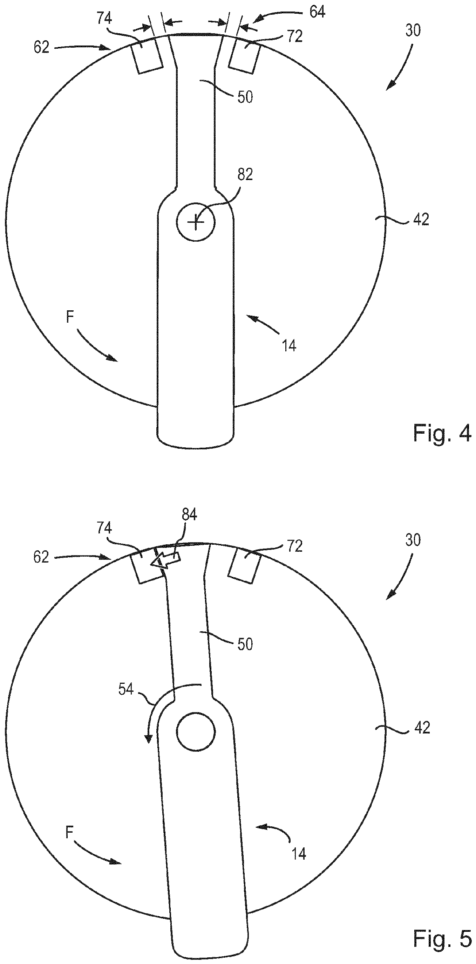

Das Stellelement 30 weist die Anschlagseinrichtung 62 auf, die bei dieser Ausführungsform auf dem Trägerelement 42 angeordnet sind. Wie im Hinblick auf die Figuren 4 und 5 besonders gut zu erkennen ist, definiert die Anschlagseinrichtung 62 einen Bewegungsbereich 64 des Abschnitts 50, und damit des Bedienelements 14 insgesamt, relativ zum Stellelement 30.

Das endoskopische Instrument 10 weist des Weiteren eine Steuereinrichtung 70 auf, die dafür ausgebildet ist, einen Messwert oder ein Messsignal bezogen auf eine Torsion des ersten Zwischenelements 40 zu erfassen und den Aktuator 32, hier ein Elektromotor, unter Berücksichtigung des Messwerts bzw. des Messsignals anzusteuern. Die elektrischen Verbindungsleitungen zwischen der Steuereinrichtung 70 und einem Messelement 56 (hier nicht gezeigt) sind zum Zwecke einer besseren Übersichtlichkeit nicht dargestellt. Es ist bevorzugt, die elektrische Verbindung zumindest abschnittsweise mittels einer Platine bereitzustellen. Die Ansteuerung des Aktuators 32 wurde bereits ausführlich für die Betriebsmöglichkeiten rein motorisch, kombiniert und rein manuell erläutert und soll hier nicht wiederholt werden.

Die Anschlagseinrichtung 62 ist hier in Umfangsrichtung des Trägerelements 42 angeordnet. Die Anschlagseinrichtung 62 weist ein erstes Anschlagselement 72 und ein zweites Anschlagselement 74 auf, die hier als Vorsprünge ausgebildet sind und zwischen denen ein Bereich des Abschnitts 50 zu liegen kommt. Die Anschlagseinrichtung 62 ist einstellbar, um den einen freien rotatorischen Bewegungsbereich 64 des Abschnitts 50 und damit das Bedienelement 14relativ zum Stellelement 30 zu variieren. Dafür weisen die Anschlagselemente 72, 74 hier jeweils eine symbolisch dargestellte Madenschraube 80 auf. Je weiter die Madenschrauben 80 aus dem jeweiligen Anschlagselement 72, 74 herausgedreht werden, desto kleiner wird der Bewegungsbereich 64.

Fig. 4 zeigt eine zweite Ausführungsform von Bedienelement 14 und Stellelement 30 in der Draufsicht, die in ihrer Funktion der ersten Ausführungsform gemäß Fig. 3 entspricht. Wird das Bedienelement 14 lediglich so weit gegenüber dem Stellelement 30 verlagert, dass der freie rotatorische Bewegungsbereich 64 um eine Verlagerungsachse 82 nicht überschritten wird, bewegt sich der Abschnitt 50 frei innerhalb des Bewegungsbereichs 64. Eine vom Benutzer auf das Bedienelement 14 aufgebrachte Kraft F überträgt sich nicht direkt auf das Stellelement 30. Vielmehr wird im Zwischenelement 40 eine Torsion erkannt, und der Aktuator 32 wird zur motorischen Bewegung des Stellelements 30, hier insbesondere des Schnurrads 44 angesteuert.

Fig. 5 zeigt die zweite Ausführungsform gemäß Fig. 4, wenn die Kraft des Benutzers so groß ist, dass der kombinierte Betrieb eintritt. Es ist zu erkennen, dass der Abschnitt 50 nun den Bewegungsbereich 64 ausgeschöpft hat und zumindest ein Teil der Kraft F, die der Benutzer über das Bedienelement 14 aufbringt, vom Abschnitt 50 auf das zweite Anschlagselement 74 der Anschlagseinrichtung 62 übertragen wird. Dies ist mittels des Pfeils 84 symbolisiert. Falls der Benutzer seine Kraft F weiter erhöht, wird diese Kraft im Wesentlichen über den Abschnitt 50 direkt auf die Anschlagseinrichtung 62 übertragen und führt nicht bzw. nur in einem geringen Ausmaß zu einer weiteren Torsion des Zwischenelements 40. Dies stellt einen Überlastschutz für das Zwischenelement 40 dar.

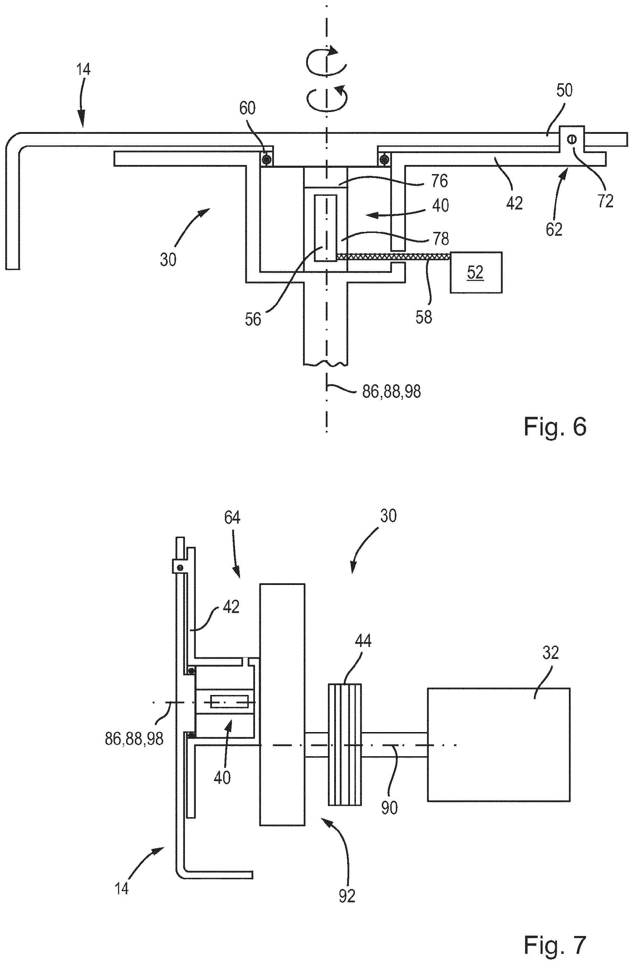

Fig. 6 zeigt Bedienelement 14, Stellelement 30 und Zwischenelement 40 einer dritten Ausführungsform in teilgeschnittener Ansicht. Es ist eine Erfassungseinrichtung 52 zum Erfassen einer Torsion des Zwischenelements 40 mit einem Messelement 56 gezeigt, wobei das Messelement 56 der Erfassungseinrichtung 52 auf dem Zwischenelement 40 angeordnet ist. Das Messelement 56 ist derart ausgebildet, dass sich bei Änderung seiner Form der elektrische Widerstand des Messelements 56 ändert. Das Messelement 56 ist hier als Dehnungsmesstreifen ausgebildet. Das Messelement 56 ist mittels einer elektrischen Verbindung 58 mit der Erfassungseinrichtung 52 verbunden.

Das Zwischenelement 40 weist hier einen ersten Bereich 76 und einen zweiten Bereich 78 auf, wobei der erste Bereich 76 aus einem anderen Material besteht als der zweite Bereich 78. Der zweite Bereich 78 ist hier im Hinblick auf die gewünschte Torsionsmessung optimiert und aus PEEK ausgeführt. Eine erste Rotationsachse 98 des Stellelements 30, eine zweite Rotationsachse 86 des Bedienelements 14 und eine Längsachse 88 des Zwischenelements 40 fallen zusammen.

Das Bedienelement 14 und das Stellelement 30 sind über ein Lager 60 miteinander gekoppelt. Das Lager ist hier als Kugellager ausgebildet. Dies ermöglicht es auf einfache Weise, dass sich das Bedienelement 14 um die erste Rotationsachse 98 des Stellelements 30 drehen lässt.

Fig. 7 zeigt Bedienelement 14, Stellelement 30, Zwischenelement 40 und Aktuator 32 einer vierten Ausführungsform in der Seitenansicht. Eine Rotationsachse 90 des Aktuators 32 ist eingezeichnet. Das Stellelement 30 weist hier ein Getriebe 92 auf. Wie in der nachfolgenden Figur noch verdeutlicht wird, handelt es sich um ein übersetztes Getriebe 92, d.h., eine Rotation des Bedienelements 14 um einen ersten Winkel führt zu einer größeren Rotation des Schnurrads 44.

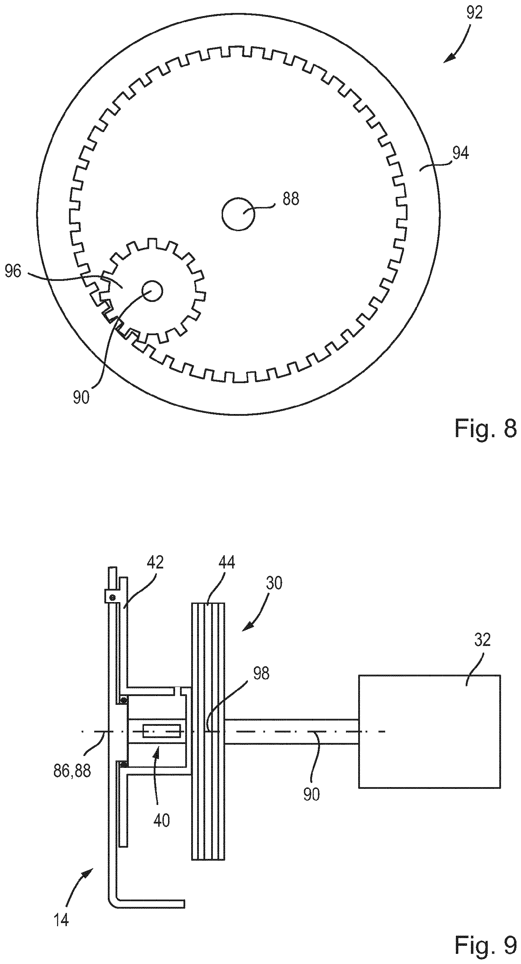

Fig. 8 zeigt eine Ausführungsform des Getriebes 92 gemäß Fig. 7. Dabei weist das Getriebe ein Innenzahnrad 94 und ein Zahnrad 96 auf. Durch eine entsprechende Wahl der Zahnräder 94, 96 kann eine gewünschte Übersetzung einfach realisiert werden.

Fig. 9 zeigt Bedienelement 14, Stellelement 30, Zwischenelement 40 und Aktuator 32 einer fünften Ausführungsform in der Seitenansicht. Bei dieser Ausgestaltung fallen die Rotationsachse 90 des Aktuators 32 und die erste Rotationsachse 98 des Stellelements 30 zusammen. Eine weitere Besonderheit ist hier, dass das Stellelement 30 als Schnurrad 44 realisiert ist.

Insgesamt wird ein endoskopisches Instrument 10 aufgezeigt, das einen rein motorischen, kombinierten und einen rein manuellen Betrieb ermöglicht. Zudem ist auch in einem Fehlerfall sichergestellt, dass der Benutzer das endoskopische Instrument 10 ohne Unterbrechung und ohne Einschränkung trotz fehlender Unterstützung durch den Aktuator 32 sicher bedienen kann.

## Patentansprüche

1. Endoskopisches Instrument (10) mit
einem Bedienstück (12) mit einem Bedienelement (14),
einem Instrumentenschaft (16) mit einem betätigbaren Abschnitt (18), wobei der Instrumentenschaft (16) mit dem Bedienstück (12) verbunden ist,
einem Stellelement (30),
einem Zugelement (34), das mit dem Stellelement (30) und dem Abschnitt (18) des Instrumentenschafts (16) mechanisch gekoppelt ist, so dass eine Verlagerung des Stellelements (30) eine Betätigung des Abschnitts (18) mittels einer Kraftübertragung über das Zugelement (34) bewirken kann,
einem Aktuator (32), der mit dem Stellelement (30) gekoppelt ist, so dass eine Betätigung des Aktuators (32) eine Verlagerung des Stellelements (30) durch Kraftübertragung vom Aktuator (32) auf das Stellelement (30) bewirken kann,
einem Zwischenelement (40), das mit dem Bedienelement (14) und dem Stellelement (30) wirkverbunden ist, so dass eine vom Benutzer auf das Bedienelement (14) ausgeübte Kraft (F) über das Zwischenelement (40) auf das Stellelement (30) übertragen werden kann, um das Stellelement (30) zu verlagern, und
einer Anschlagseinrichtung (62), die einen freien rotatorischen Bewegungsbereich (66) des Bedienelements (14) relativ zum Stellelement (30) definiert,
das Instrument **gekennzeichnet durch** eine Erfassungseinrichtung (52) zum Erfassen einer Torsion des Zwischenelements (40) und eine Steuereinrichtung (70), die dafür ausgebildet ist, einen Messwert der Torsion des Zwischenelements (40) aufzunehmen und den Aktuator (32) unter Berücksichtigung des Messwerts anzusteuern.

2. Endoskopisches Instrument nach Anspruch 1, wobei ein Messelement (56) der Erfassungseinrichtung (52) auf dem Zwischenelement (40) angeordnet ist.

3. Endoskopisches Instrument nach Anspruch 1 oder 2, wobei die Erfassungseinrichtung (52) ein Messelement (56) aufweist, das derart ausgebildet ist, dass sich bei Änderung einer Form des Messelements (56) der elektrische Widerstand des Messelements (56) ändert.

4. Endoskopisches Instrument nach einem der vorhergehenden Ansprüche, wobei die Erfassungseinrichtung (52) ein Messelement (56) aufweist, das als Dehnungsmesstreifen ausgebildet ist.

5. Endoskopisches Instrument nach einem der vorhergehenden Ansprüche, wobei die Erfassungseinrichtung (52) mindestens zwei Messelemente (56) aufweist, die derart angeordnet sind, dass in der Erfassungseinrichtung (52) eine Kompensation von mindestens einem Störeinfluss ausgewählt aus der Gruppe bestehend aus Biegung, Längsstreckung und Temperatur ermöglicht ist.

6. Endoskopisches Instrument nach einem der vorhergehenden Ansprüche, wobei das Zwischenelement (40) zumindest einen ersten Bereich (76) und einen zweiten Bereich (78) aufweist, wobei der erste Bereich (76) aus einem anderen Material besteht als der zweite Bereich (78).

7. Endoskopisches Instrument nach einem der vorhergehenden Ansprüche, wobei das Zwischenelement aus einem anderen Material besteht als das Bedienelement (14) und/oder das Stellelement (30).

8. Endoskopisches Instrument nach einem der vorhergehenden Ansprüche, wobei die Anschlagseinrichtung (62) einstellbar ist, um den Bewegungsbereich (66) zu variieren.

9. Endoskopisches Instrument nach einem der vorhergehenden Ansprüche, wobei das Stellelement (30) ein Getriebe (92) aufweist, insbesondere ein Getriebe (92) mit einem positiven Übersetzungsverhältnis.

10. Endoskopisches Instrument nach einem der vorhergehenden Ansprüche, wobei eine erste Rotationsachse (98) des Stellelements (30), eine zweite Rotationsachse (86) des Bedienelements (14) und eine Längsachse (88) des Zwischenelements (40) zusammenfallen.

11. Endoskopisches Instrument nach einem der vorhergehenden Ansprüche, wobei eine erste Rotationsachse (98) des Stellelements (30) und eine dritte Rotationsachse (90) des Aktuators (32) zusammenfallen.

12. Endoskopisches Instrument nach einem der vorhergehenden Ansprüche, wobei das Stellelement (30) ein zumindest in etwa kreisförmiges Trägerelement (42) aufweist, auf dem die Anschlagseinrichtung (62) zumindest in etwa in Umfangsrichtung des Trägerelements (42) angeordnet ist.

## Claims

1. Endoscopic instrument (10) comprising
a control section (12) with a control element (14),
an instrument shaft (16) with an actuatable portion (18), wherein the instrument shaft (16) is connected to the control section (12),
an adjustment element (30),
a pull element (34), which is mechanically coupled to the adjustment element (30) and to the portion (18) of the instrument shaft (16), such that a movement of the adjustment element (30) can cause an actuation of the portion (18) by means of a force transmission via the pull element (34),
an actuator (32), which is coupled to the adjustment element (30), such that an actuation of the actuator (32) can cause a movement of the adjustment element (30) by force transmission from the actuator (32) to the adjustment element (30),
an intermediate element (40), which is operatively connected to the control element (14) and to the adjustment element (30) such that a force (F) applied to the control element (14) by the user can be transmitted to the adjustment element (30) via the intermediate element (40) in order to move the adjustment element (30),
an abutment device (62), which defines a free rotatory movement range (66) of the control element (14) relative to the adjustment element (30)
the instrument **characterized by** a detection device (52) for detecting a torsion of the intermediate element (40), and a controller (70) adapted to detect a measured value of the torsion of the intermediate element (40) and to drive the actuator (32) taking into account the measured value.

2. Endoscopic instrument of claim 1, wherein a measurement element (56) of the detection device (52) is arranged on the intermediate element (40).

3. Endoscopic instrument of claim 1 or 2, wherein the detection device (52) has a measurement element (56) which is adapted in such a way that the electrical resistance of the measurement element (56) changes in the event of a change of shape of the measurement element (56).

4. Endoscopic instrument of any of the preceding claims, wherein the detection device (52) has a measurement element (56) adapted as a strain gauge.

5. Endoscopic instrument of any of the preceding claims, wherein the detection device (52) has at least two measurement elements (56) which are arranged in such a way that, in the detection device (52), a compensation is permitted for at least one interference chosen from the group consisting of flexion, longitudinal extension and temperature.

6. Endoscopic instrument of any of the preceding claims, wherein the intermediate element (40) has at least a first area (76) and a second area (78), wherein the first area (76) is made from a different material than the second area (78).

7. Endoscopic instrument of any of the preceding claims, wherein the intermediate element is made from a different material than the control element (14) and/or the adjustment element (30).

8. Endoscopic instrument of any of the preceding claims, wherein the abutment device (62) is adjustable, in order to vary the movement range (66).

9. Endoscopic instrument of any of the preceding claims, wherein the adjustment element (30) has a gear (92), in particular a gear (92) with a positive transmission ratio.

10. Endoscopic instrument of any of the preceding claims, wherein a first rotation axis (98) of the adjustment element (30), a second rotation axis (86) of the control element (14), and a longitudinal axis (88) of the intermediate element (40) coincide.

11. Endoscopic instrument of any of the preceding claims, wherein a first rotation axis (98) of the adjustment element (30) and a third rotation axis (90) of the actuator (32) coincide.

12. Endoscopic instrument of any of the preceding claims, wherein the adjustment element (30) has an at least approximately circular support element (42), on which the abutment device (62) is arranged at least approximately in the circumferential direction of the support element (42).

## Revendications

1. Instrument endoscopique (10), avec
une pièce de commande (12) avec un élément de commande (14),
une tige d'instrument (16) avec un tronçon (18) pouvant être actionné, la tige d'instrument (16) étant raccordée à la pièce de commande (12),
un élément de réglage (30),
un élément de traction (34) qui est couplé mécaniquement à l'élément de réglage (30) et au tronçon (18) de la tige d'instrument (16) de telle sorte qu'un déplacement de l'élément de réglage (30) peut provoquer un actionnement du tronçon (18) au moyen d'une transmission de force par le biais de l'élément de traction (34),
un actionneur (32) qui est couplé à l'élément de réglage (30) de telle sorte qu'un actionnement de l'actionneur (32) peut provoquer un déplacement de l'élément de réglage (30) par transmission de force de l'actionneur (32) vers l'élément de réglage (30),
un élément intermédiaire (40) qui est en liaison opérationnelle avec l'élément de commande (14) et l'élément de réglage (30) de telle sorte qu'une force (F) exercée par l'utilisateur sur l'élément de commande (14) peut être transmise à l'élément de réglage (30) par le biais de l'élément intermédiaire (40) pour déplacer l'élément de réglage (30), et
un dispositif de butée (62) qui définit une zone de mouvement (66) rotatif libre de l'élément de commande (14) relativement à l'élément de réglage (30),
l'instrument étant **caractérisé par** un dispositif de détection (52) destiné à détecter une torsion de l'élément intermédiaire (40) et par un dispositif de commande (70) qui est constitué pour enregistrer une valeur de mesure de la torsion de l'élément intermédiaire (40) et pour piloter l'actionneur (32) en prenant en compte la valeur de mesure.

2. Instrument endoscopique selon la revendication 1, un élément de mesure (56) du dispositif de détection (52) étant disposé sur l'élément intermédiaire (40).

3. Instrument endoscopique selon la revendication 1 ou 2, le dispositif de détection (52) présentant un élément de mesure (56) qui est constitué de telle sorte que, lors de la variation d'une forme de l'élément de mesure (56), la résistance électrique de l'élément de mesure (56) varie.

4. Instrument endoscopique selon l'une des revendications précédentes, le dispositif de détection (52) présentant un élément de mesure (56) qui est constitué en tant que jauge extensométrique.

5. Instrument endoscopique selon l'une des revendications précédentes, le dispositif de détection (52) présentant au moins deux éléments de mesure (56) qui sont disposés de telle sorte que, dans le dispositif de détection (52), une compensation d'au moins une influence perturbatrice sélectionnée dans le groupe composé de la flexion, de l'étirement et de la température est rendue possible.

6. Instrument endoscopique selon l'une des revendications précédentes, l'élément intermédiaire (40) présentant au moins une première zone (76) et une deuxième zone (78), la première zone (76) étant composée d'un matériau différent de celui de la deuxième zone (78).

7. Instrument endoscopique selon l'une des revendications précédentes, l'élément intermédiaire étant composé d'un matériau différent de celui de l'élément de commande (14) et/ou de l'élément de réglage (30).

8. Instrument endoscopique selon l'une des revendications précédentes, le dispositif de butée (62) étant réglable pour faire varier la zone de mouvement (66).

9. Instrument endoscopique selon l'une des revendications précédentes, l'élément de réglage (30) présentant un engrenage (92), en particulier un engrenage (92) avec un rapport de transmission positif.

10. Instrument endoscopique selon l'une des revendications précédentes, dans lequel un premier axe de rotation (98) de l'élément de réglage (30), un deuxième axe de rotation (86) de l'élément de commande (14) et un axe longitudinal (88) de l'élément intermédiaire (40) coïncident.

11. Instrument endoscopique selon l'une des revendications précédentes, dans lequel un premier axe de rotation (98) de l'élément de réglage (30) et un troisième axe de rotation (90) de l'actionneur (32) coïncident.

12. Instrument endoscopique selon l'une des revendications précédentes, l'élément de réglage (30) présentant un élément de support (42) au moins approximativement circulaire sur lequel le dispositif de butée (62) est disposé au moins approximativement dans la direction circonférentielle de l'élément de support (42).
